# EUROPEAN PATENT SPECIFICATION

(11) **EP 1 583 490 B1**
(45) Date of publication and mention of the grant of the patent: **10.12.2008**
(21) Application number: 03703860.1
(22) Date of filing: 16.01.2003
(51) Int. Cl.: A61F 2/44

(54) **HYDROGEL-BASED PROSTHETIC DEVICE FOR REPLACING AT LEAST A PART OF THE NUCLEUS OF A SPINAL DISC**
AUF HYDROGEL BASIERENDE PROTHESE ZUM MINDESTENS TEILWEISEN ERSATZ DES NUKLEUS EINER BANDSCHEIBE
DISPOSITIF PROTHETIQUE A BASE D'HYDROGEL DESTINE AU REMPLACEMENT D'UNE PARTIE AU MOINS DU NOYAU D'UN DISQUE INTERVERTEBRAL

(43) Date of publication of application: 12.10.2005
(60) Divisional application: 07021290.7
(73) Proprietor: Replication Medical, INC., New Brunswick, NJ 08901 (US)
(72) Inventor: STOY, Vladimir, A., Princeton, NJ 08540 (US)
(74) Representative: Laufhütte, Dieter
(86) International application number: PCT/US2003/001434
(87) International publication number: WO 2004/064693

(56) References cited:
- EP-A- 0 356 112
- EP-A- 0 505 634
- WO-A-00/64385
- WO-A-02/076336
- US-A- 5 534 028
- US-A- 5 674 295

## Description

### BACKGROUND OF THE INVENTION ,

### 1. Field Of The Invention

The present invention is directed to a hydrogel-based prosthetic device to replace all or part of the nucleus of a spinal disc after it has been surgically removed. More specifically, the present invention is directed to such a device which contains a uniquely advantageous combination of differing structural compositions and geometries.

### 2. Description of Related Art

The spinal disc is a cartiligeous spinal joint which allows the bending and rotation of the spine. Damage to the spinal disc leads to a dysfunction of the spine, serious pain, and often to a long-term disability of the patient. A typical problem consists of the spinal disc bulging out or herniating so that the nucleus becomes extruded, which then causes a compression of an adjacent nerve and an inflammatory reaction. Presently, it is typical to immobilize the joint by fusing the neighboring vertebrae using various instruments and techniques. It is also common to remove the nucleus or its part in a procedure called a laminectomy.

All present surgical interventions, whether laminectomy or fusion of adjacent vertebrae, lower the functionality of the spine in some way. For that reason it is desirable to try to develop a prosthetic for the spinal disc or its part. This is, however, extremely difficult. The spine is a very complex part of the body and its proper function is dependent on proper coordination of the function of all the parts, including the spinal discs. The system needs to withstand complex stresses, including various angles of bending, pressure, shear, and twisting. The spinal disc must also function as a shock and vibration absorber. And finally, a spinal disc must allow the transport of the nutrients and metabolic products needed for its health and survival. These complex requirements elucidate the complex structure of the spinal disc.

The spinal disc is made of a hydrogel-like core called Nucleus Pulposus (NP), and an outer sheath called Annulus Fibrosus (AF). The AF consists mainly of collagen fibers which are organized into many criss-crossed layers somewhat like layers in automotive tires. This configuration ensures significant resistance against radial stress and inner over-pressure, while allowing significant deformation during twisting and bending. The NP is located within the AF. The NP is a hydrogel-like composite made ofproteoglycans and collagen, with a uniform water content of more than 90% of total mass. Its composition is similar to the AF except it has a lower collagen content and a higher content of proteoglycans and water. Aside from that, the NP is isotropic, while the AF is significantly anisotropic. The NP is connected to the AF, and the transition between these two bodies is gradual.

The areas of adjacent vertebrate bodies can also be thought of as parts of the intra-vertebral joint system. These areas are covered with cartilage consisting of a collagen matrix filled with glycoprotein and water. Just like the NP and the AF, this cartilage is a living tissue that contains approximately 2-5% of live cells needed to renew the cartilage. The collagen fibrilles of the AF are attached to the vertebrate area cartilage. The NP is attached to the AF, but not to the vertebrate area cartilage. This arrangement is important for the mobility and proper function of the intra-vertebral joint. The function of the spinal disc can be compared to the function of a tire in a car. In this case the "tire" itself is made of the AF, while the NP functions like the compressed air in the tire. The NP has viscoelastic mechanometric characteristics. Aside from that it is, like air, capable of changing its volume with a changing load. This is achieved by the change of the water content due to outside pressure. Resistance produced by the nucleus to the reduction of water content by mechanical pressure ("wringing") is called "swelling pressure". Such pressure is produced by the partially dehydrated NP gel, which is trying to absorb water to regain equilibrium and increase its volume. Swelling pressure is also a key point in the function of the intra-vertebral spinal disc. When the axial pressure load increases, part of the liquid is expelled from the nucleus, which increases the swelling pressure (the concentration of the polymer increases). The process will stop only when equilibrium is reached and the axial pressure equals the swelling pressure. In this way, the *NP* is able to balance out and apportion the pressure in the spine, and it does so especially by transferring axial load into radial load, which is then captured by the AF. Furthermore, the changes in swelling are the driving force of the transportation of metabolites and nutrients, without which the NP tissues could not survive in the long term.

From the above-stated facts it is obvious, that the construction of a fully functional prosthesis is extremely difficult. Most prosthetic devices suggested to date are strictly mechanical, and they mimic only some functions of the disc. An example of such are constructions described in US patents by Froning (3,875,595), Kuntz (4,349,921), Shepperd (4,863,476), Olerud (5,053,034), Bryan et al. (5,674,269 and 5,865,846), Yuan et al. (5,676,701) and Serhan, et al. (5,834,094).

A more perfect prosthetic with a truer simulation of the disk function was suggested in a US Patent No. 4,911,718 "Functional and Biocompatible Intervertebral Spacer" (Lee et al, 1990), describing a composite construction of the prosthetic of the disc using a biocompatible elastomer, reinforced by fibers which mimic the function of collagen fibers in a natural spinal disc. The main disadvantage of this solution, which is common to all full spinal disc replacements, remains a complicated surgical procedure, which translates into a high cost, and a high risk to the patient.

In many cases, only the NP (or even a portion of the NP) can be replaced to restore function. The replacement of a missing NP will keep the AF in a necessary state of tension, which will also enable its proper mechanical function. It is also necessary to replace the function of the original NP in regards to nutrient and metabolic waste transport, since without this feature the remaining living tissues of the spinal disc cannot survive. For that reason, the NP substitute must be made of a hydrogel with a sufficient swelling pressure and a capability of hydraulic transport of fluids.

A hydrogel substitute for the NP was first suggested by Bao et a1. in the US Patent No. 5,047,055. Bao describes a hydrogel prosthesis of the nucleus, whose shape and size corresponds to the removed disc nucleus when the prosthesis is fully swollen. According to the requirements stated in the patent, the hydrogel used in a fully swollen state must have a water content of at least 30% by weight, and a pressure strength of at least 4 MN.m⁻². This high strength must be achieved even at full swelling in water and during a high water content (Bao suggests 70% to 90% by weight as optimum). This high strength is apparently requested in order to prevent isotropic extrusion of the material implanted into the damaged and weakened AF. The selection of hydrogels fulfilling this requirement is narrow, however.

Furthermore, Bao teaches to implant his prosthetic in a partially dehydrated state when the dimensions are smaller and the device can be inserted through a smaller opening. After implantation, the prosthetic will grow to its full size by absorbing bodily fluids. It is necessary to note, however, that the dehydration prior to implantation and rehydration after implantation are isotropic, i.e., all dimensions change at the same rate. This can be seen as a significant disadvantage of the Bao concept. During implantation the implant will try to expand equally in all directions, but it will expand most in the direction of the least resistance. Therefore it will expand the least in the axial direction, where expansion is most needed (so that the separation of the vertebrae is the highest), and it will expand the most in the radial direction, where the expansion is least desirable; especially in places where the AF is weakened or even missing. For this reason Bao was forced to suggest an implant which is in its fully swollen state exactly the size of the cavity created by the removal of the NP or its part. This presents a significant obstacle, however, and also means a lowered function of the implant, which will then will have a zero swelling pressure at a fully swollen state.

Another disadvantage, stemming from the isotropic characteristics of the material, is its radial bulging under axial pressure. This bulging will be the greatest in the direction of the least resistance, i.e., in the direction where the AF is weakened or damaged. The implant can also have the tendency to slowly change shape and herniate when subject to permanent and long-term pressure. The high strength and modulus requirements which Bao states are apparently governed by the effort to prevent such undesirable deformation.

Some of the stated deficiencies were later remedied in the following US Patent No. 5,192,326 (Bao et al). In this case the prosthetic is formed by hydrogel spheres placed within an elastic, semi-permeable sack or wrap. The porous wrap, in its fully unfurled state, has the shape and size of the cavity created by the removal of the NP or its part. The size of hydrogel spheres is at least three times the size of the size of the pore, therefore they cannot escape out of the wrap. The hydrogel can contain up to 99% fluid by weight in its fully swollen state, and its strength doesn't need to be as high as in the previous case since the strength of the implant is given by the strength of the wrap and not of the hydrogel. The volume of the fully swollen hydrogel cannot be larger than the size of the cavity created by the removal of the NP, since the full swelling is prevented by the resistance of the wrap against further expansion.

A similar implant is described by Ray et al. in US Patent No. 4,772,287. Ray describes an implant into NP, consisting of two cylindrical bladders filled with a fluid. The bladders are enclosed in a fibrous wrap.

In a US Patent No. 4,904,260, Ray describes an improvement upon the previous invention, which relies on the bladders being semi-permeable, and the liquid in it contains a substance with a therapeutic effect. This substance is capable of slowly diffusing from the prosthetic into the surrounding tissue.

A further improvement is described by the same author in a US Patent No. 5,674,295. Here, the bladders filled with liquid are replaced by cylindrical hydrogel bodies. The solid fibrous wrap allows for higher swelling in the axial direction and prevents excessive swelling in the radial direction, thus protecting the AF from bulging due to the swelling pressure of the implant.

This invention is further modified in a US Patent No. 5,824,093 where the hydrogel bodies have an oval rather than a circular cross-section, and their wrap is constructed so that their general shape is maintained even during full swelling and loading. The implants described by Ray are not an actual replacement of the NP, because they have significantly different shape and characteristics. More specifically, this is a device for a partial fusion and partial immobilization of the spinal disc, rather than the restoration of its function.

WO 00/64385 discloses a prosthetic nucleus for implantation which is made of hydrogel and expands after implantation. It can be constructed with a composite construction, utilizing two or more materials. This prothesis may have multiple internal and/or external surfaces that may be connected and/or reinforced.

EP 0 505 634 A1 discloses a prothesis, more specifically an artificial bone connection prothesis used for the replacement of damaged bones, an articular cartilage such as a coax and a knee joint, and a spine's intervertebral disc.

The above overview indicates that a fully optimal NP prosthetic was not yet described.

### SUMMARY OF THE PRESENT INVENTION

The present invention is a prosthesis for replacement of at least a part of the nucleus of a intravertebral disc. The prosthetic device is composed of at least two essentially parallel soft layers of an elastically deformable hydrogel and at least one rigid layer, the rigid layer having less compressibility than the soft layers, being adjacent to the soft layers, parallel to them, and firmly attached to them. In some embodiments, the soft layers have the same thickness and composition. Typically, the prosthesis has more than one rigid layer and these rigid layers have the same thickness and composition. The number of soft layers is usually one more than the number of rigid layers, with, e.g., at least three soft layers. The invention also includes a method of prosthesis production, which involves prefabricating soft and rigid layers; stacking at least two prefabricated soft and at least one prefabricated rigid layer in a parallel fashion into their final form, and, permitting the layers to firmly connect to one another by mutual interaction. In this method, at least one type of the layers is prefabricated in the dehydrated state, which is done by dehydrating stretched foil in an apparatus preventing its contraction and thereby decreasing its area. There is at least partial dehydration of the prosthesis under pressure applied in a direction tangent to the planes of the layers. In preferred embodiments, while in the state of almost full dehydration, is sterilized using ionizing radiation or a gaseous chemical agent, after which it is partially rehydrated within the sterile wrapper using water vapor.

### BRIEF DESCRIPTION OF THE DRAWINGS

The present invention is more fully understood when the invention is taken in conjunction with the appended drawings, wherein:
FIGS. 1 and 2 show side cut views of one embodiment of a prosthetic device according to the present invention in its relaxed and compressed states, respectively. Thus, device 1 has an outer encasement 3, with a top 5, sides 7 and 9, and bottom 11, formed of a flexible skin and contains soft layers 13, 15, 17 and 19, and rigid layers 21,23 and 25.
FIG. 3 is a schematic depiction of one embodiment of a spinal disc implant according to the present invention.

### DETAILED DESCRIPTION OF THE PRESENT INVENTION

The stated inadequacies are removed by a prosthesis of the nucleus or its part of the intravertebral disc according to this invention as follows:

In accordance with the present invention, a spinal disc implant is provided that is made of layers of more rigid and less rigid materials. The unique physical and chemical properties of the implant allow it to be inserted into the intervertebral disc space in a compressed, relatively dehydrated state. After implantation, the implant expands anisotropically through the absorption of body fluids. The implant's unique construction provides a relatively large amount of swelling in the axial direction and a relatively limited amount of swelling in the radial direction. It should be understood that the terms "prosthetic" and "implant" are used interchangeably herein.

The invention is based, in part, on incorporation of hydrogels. In certain embodiments, an implant is constructed of parallel or substantially parallel flexible or soft layers of an elastically deformable hydrogel, which may be of equal thickness, and one or more relatively rigid layers as compared to the soft layers, which may also be of equal thickness and which are adjacent to the soft layers and are parallel or substantially parallel to them. It should be understood that, as used herein, "substantially parallel" is intended to mean that the layers can be completely parallel or they can be nearly, but not wholly, parallel. The layers may be firmly attached to one another by mutual cross-linking of both layers, for example, by using the physical interaction between polymer chains or and adhesive interface, which is capable of reacting with both neighboring strata. Accordingly, a structure with alternating layers is provided, which can be more or less subject to tensile deformation. The terms "soft" and "rigid" are used herein to describe the relative difference between layers, i.e., more or less rigid. Thus, the "rigid" layers may still be deformable; they are just more resistant to deformation as compared to the "soft" layers.

An advantageous arrangement will contain at least three soft layers. The combination of soft and relatively rigid layers also allows variations where the number of relatively rigid layers is one less than the soft layers, and vice versa. If the number of soft layers is larger by one, then the contact between the prosthetic and the cartilage of the vertebra is enabled by the softer hydrogel with a higher water content. This has a number of advantages, for example a gentler contact with the cartilages, and better hydraulic transport of water in and out of the prosthetic. By the same token, the terminus of the prosthetic is preferably secured by encapsulation of the layered nucleus in a soft hydrogel cover.

Similarly it is possible to use a wrap made of a more rigid material, possibly even a wrap made of a textile material. Such a wrap can favorably effect the mechanical durability of the prosthetic and its resistance to herniation in case of a weakened AF.

If the number of less-deformable layers is one more then the others, then the contact with the vertebral cartilage is formed by the more rigid, less yielding and less slippery surface. This can be an advantage especially in cases where the AF is weakened, and it is necessary to ensure increased friction between the prosthetic and the vertebrae. It is of course also possible to arrange a contact of one vertebra with a soft surface, and the other vertebra with a hard surface, depending on the state of the vertebrae and the sheath of the disk. In this case the number of soft and hard layers would be the same.

According to this invention, the prosthetic can have different layers of different thicknesses, which would aid the design of various profiles under load and various distributions of pressure in the prosthesis. In most cases it is advantageous for all soft layers to be of an equal thickness, which will result in minimal bulging for a given number of layers under load. The uniformity of each type of layers regarding the thickness and composition will also aid easy production.

An advantageous part of the construction are also cavities or channels in the hydrogel, which positively affect the ability of the prosthesis to deform under low loads and support the transportation of fluids. In most cases it is advantageous to arrange the channels of an appropriate diameter and number vertically, that coaxially with the axis of the spine. Such channels mutually and advantageously connect a certain number of parallel layers, as well the surface of the prosthetic.

The soft, less rigid layer is formed by high-strength, high water content hydrogels. An example of such hydrogels is polyvinylalcohol (PVA), physically cross-linked by partial crystallization of the chain. Such hydrogels are described, for example, in US Patent 4,663,358. Another example are hydrogels based on segmented polyurethanes or polyureas, an example of which is described in US Patent 5,688,855. In principle, polypeptide or polysaccharide hydrogels could be used, an example of which is agarose or cross-linked hyaluronic acid, even though biodegradation often limits the lifetime of such implants. According to this invention, very advantageous hydrogels for the prosthetic are based on partially hydrolyzed or aminolyzed polyacrylonitrile (PAN), an example of which is described in US Patents 4,943,618, 4,107,121, and 5,252,692 .

The hydrogel in the soft layer has an advantageous equilibrium water content higher than about 75% by weight and at full swelling, and if possible higher than about 85% by weight. Most advantageous are hydrogels with a water content over about 90% by weight to about 95 or 96% by weight, being similar to a water content of the natural nucleus of the disk. Such hydrogels do not need to posseses high strength at full swelling by water since in their functional state they are significantly dehydrated both osmotically and through mechanical pressure. This limited swelling in the functional state is entirely essential to the function of the prosthetic. Therefore, the size of the prosthetic at full swelling by water is significantly higher than the size of the original nucleus, and/or higher than the size of the cavity created by the partial or entire removal of the original nucleus. For the same reason the shape of the fully hydrated (swollen) prosthetic in water is different than the shape into which the prosthetic is inserted in a dehydrated and deformed state. On the other hand, these hydrogels should have a sufficient hydraulic permeability for water to allow dehydration and rehydration via pressure. Further they must possess sufficient biocompatibility, and especially they must not release any harmful substances. They must also be sufficiently stable in their implanted state to guarantee a sufficiently long function of the prosthetic (which does not necessarily mean that they need to last to the end of the life of the patient -these prosthetics are useful even if they are used for a temporary improvement of function of the spinal disc).

The rigid layer must possess the characteristics of sufficient stability, compatibility, and other qualities important for implants in general such as biocompatability, but also must possess a sufficient tensile elastic modulus. It can be made of various polymers, such as polyethyleneterephthalate (Dacron), polyamide, polyurethane, polyureas, acrylic and methacrylic polymers, expanded polytetrafluoroethylene (Goretex), graphite, rare metal alloys, etc. These materials can be used either alone, or in a composite form in combination with elastomers or hydrogels. Especially advantageous are woven, perforated, or porous formats of these materials which will allow solid anchoring of the soft layer.

The rigid layer will work well if it will have an easy tensile deformation to a certain deformation point, at which time the resistance to further deformation will increase sharply.

Such behavior can be seen, for example, in polymers crystallizing under tensile stress, such as natural rubber, or knits and other textile configurations. This type of mechanical behavior allows the construction of prosthetics with optimal mechanical characteristics. In one embodiment, the implant contains a reinforcing member which assists in limiting or completely limits radial expansion. Thus, in one embodiment, the rigid layer includes a woven or non-woven textile (for example, a Dacron net) reinforcing member embedded in a relatively rigid hydrogel of a similar or identical type as the one used in the soft layer, but the two would have different levels of water content. This combination ensures excellent adhesion between the soft and the rigid layer via mutual cross-linking which can be facilitated through physical interactions. It also ensures proper anchoring of the textile insert within the hydrogel. This also ensures proper pressure distribution between the hydrogel and the textile insert. "Softness" and "rigidity" of both types of layers is relative. The soft layer is typical in exhibiting a relative extension under tensile stress larger than about 200% and preferably even exceeding about 400% in a fully swollen state. The rigid layer has a reversible relative deformation lower than about 100%.

The invention also includes the adjustment of the implant so it is ready for implantation. A prosthetic in this state is made smaller through dehydration, and plastified using an appropriate, physiologically harmless softening agent such as water or glycerol. This is best be done at a concentration where the softening temperature (the transition between the vitreous and the deformed state) is higher than about -5°C (and even better, above the room temperature) and lower than about 40°C, and preferably lower than bodily temperature. This temperature transition can be characterized by a softening temperature or a glass transition temperature Tg (the values for the same composition vary somewhat according to the method used. According to the invention, a preferred method is described in US Patent 4,731,079, Example 1). The prosthetic in the described state makes the implantation possible in the deformed state in a shape (e.g., rolled up like a burrito), which is then inserted through an opening of a minimum size (such as a small channel). Once the insertion is completed, the implant will unfurl relatively quickly and assume a flat and properly oriented shape, in which it swells in a predominantly axial direction.

Another characteristic of the invention involves swelling or expansion through hydration that occurs mainly in one direction. This can be achieved by dehydrating the prosthetic during the production process by introducing a pressure deformation in the axial direction (in a direction tangent to the surface of the layers and along a longitudinal axis) in a way which will maintain the contour and area of the layers while decreasing the thickness of the layers in a ratio corresponding to the volume change caused by dehydration. The hydration of the prosthetic then causes radial shrinking, along an axis transverse to the longitudinal axis which compensates for swelling in the radial direction. Radial shrinking or expansion during swelling can be controlled within rather wide criteria. As a result, the expansion during hydration is focused mostly on the desirable axial direction, along the longitudinal axis and is limited or eliminated in the radial direction along the transverse axis. Any increase or swelling in the radial direction (the transverse axis) is preferably less than about 25% more than the width of the implant in the dehydrated state.

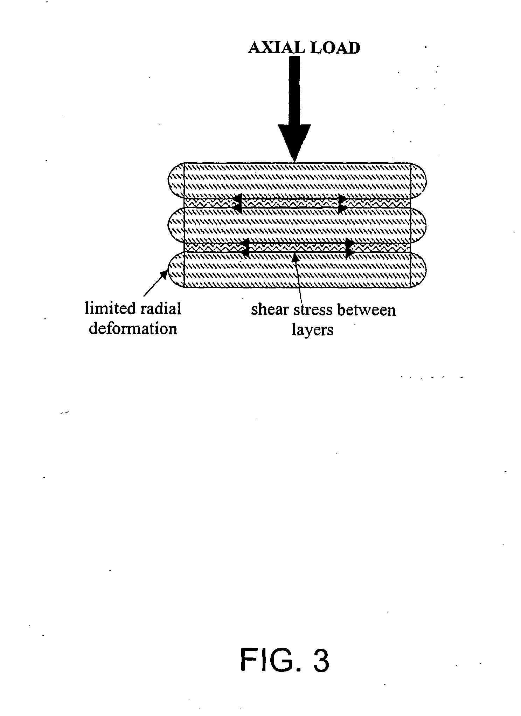

The layered construction of the invention is especially advantageous for radial deformation, which can be introduced either during drying or at a temperature above the softening point and a subsequent chilling under pressure. This arrangement means that the ability of the prosthetic to be deformed under pressure is significantly different for different directions. The configuration of alternating substantially parallel layers has a number of advantages as compared to other support element configurations. The first and essential advantage is the limitation of radial bulging caused by axial pressure. Pressure tangent to the planes of the layers (normal or axial pressure) is partially transformed into a shear stress among the layers. See FIG. 3. Soft layers are elastically deformed, but the increase of their cross-section is limited by low deformability of the rigid layers. This also limits the overall radial bulging of the prosthetic. This ensures a resistance against the herniation of the prosthetic in a place of a weakened AF. If the prosthetic is suddenly put under a pressure load, individual soft layers will bulge radially. The water content in the layers will drop over time, however, which will cause a lessening of the radial bulging. Another advantage of this configuration is a basic lack of limitation of torsional deformation or deformation during bending. Other types of rigid support limiting radial bulging tend to also limit the torsional deformation during bending.

Prior to hydration, the fully dehydrated or partially dehydrated spinal disc implant is inserted into the cavity, which was originally filled by the natural disk nucleus that was subsequently removed using surgical or biochemical methods. The prosthetic is oriented in such a way, in which the planes of all the layers are oriented more or less tangent to the axis of the spine, i.e. tangent to the vector of pressure loading (axial pressure). The layer configuration limits radial deformation of the soft layers and thus the bulging of the prosthetic and its herniation from the disk. This radial bulging will be even smaller with a decreasing height of individual soft layers at rest, i.e. with an increased overall number of layers.

Radial deformation is limited by the axial pressure being converted to shear stress between the individual layers. Maximum permissible shear stress determines the maximum axial pressure loading, which will increase with the number of layers, the strength of the layers, and the strength of bonding between individual layers.

This configuration has an advantage as compared to other composite constructions in that the dehydration of the hydrogel components via axial pressure is minimally limited. This dehydration is necessary for the function of the prosthetic, since its cyclical dehydration and rehydration supplies nutrients to the living tissue of the disc. Another advantage of the invention is the maximal flattening during dehydration, which is important for the implantation of the prosthetic through the smallest opening possible. The prosthesis is prepared for implantation in the following way: the dehydrated, flattened disc is sterilized using any suitable sterilization technique known to those skilled in the art, e.g., by using ionizing radiation or a gaseous chemical agent, and the disc then is partially rehydrated within a sterile wrapper. Thus prepared disc is adjusted by being rolled up into a shape of a burrito, omelet or other suitable shape. Another important advantage is the fact that the hydration (swelling) expansion happens essentially in the axial direction without exhibiting radial expansion which might damage surviving tissues (annulus fibrosus) and cause the herniation of the nucleus. And finally, the construction of the prosthetic according to this invention leads to an optimal pressure distribution within the prosthetic, allowing it to handle even extreme loads common for the spinal environment.

Another aspect of this invention is the manufacturing method of the prosthetic using layers of hydrogels. The main problem is achieving complete adhesion among the layers and the prevention of deformation of the prosthetic as a result of uneven expansion or contraction of individual layers during production. Although the implant can be manufactured by use of conventional techniques known to those skilled in the art, a preferred manufacturing method of this invention is based on at least one type of the layers being previously prepared in a state of controlled dehydration, and is oriented so that the swelling expansion is demonstrated as a basic increase in thickness (rather than the increase of contour and surface area dimensions). This method of dehydration is performed in a solution which prevents the contraction of the surface and thus the decrease of the surface area.

In one embodiment, the manufacturing of a prosthetic based on a hydrogel sandwich structure can be easily realized by using three components, such as hydrogels of the AQUACRYL type (made by GEL-MED International) and polyester surgical netting (made by VUP a.s. Brno):
Hydrophilic polymer with a higher water content, such as AQUACRYL 90 (AQC 90)
Hydrophilic polymer with a lower water content, such as AQUACRYL 80 (AQC 80)
Polyester surgical netting (PES)

Each of these components has its own specific function in the resulting prosthetic:
AQC 90 forms the basis of the prosthetic, acting as the main connecting element of its individual parts, it determines its basic mechanical properties, and simultaneously it ensures good biocompatibility with the surrounding tissues
AQC 80 improves the mechanical properties of the prosthetic (especially it increases its pressure strength modulus) and simultaneously it ensures good adhesion of AQC 90 to PES
PES forms a reinforcement whose main function is to prevent the deformation of the prosthetic in a way which would allow it to herniate out of the intravertebral space.

The combination of characteristics of individual components in the sandwich structure yields a prosthetic whose mechanical properties (especially its behavior during dynamic strain under pressure) mimics the characteristics of the actual Nucleus Pulposus as closely as possible, and which simultaneously yields good biocompatibility with surrounding tissues.

Thus in one embodiment, a polymer such as Aquacryl 90 is dissolved in a suitable solvent such as a 55% solution of sodium thiosulfate in water to form a viscous solution. The viscous solution is poured into a porous mold having, e.g., a cylindrical shape. The pores should be sufficiently small as to not permit the polymer to diffuse or leak out of the mold. The more rigid layer is then placed on top of the viscous solution. The more rigid layer may be a preformed hydrogel such as Aquacryl 80 which was solvent cast, washed, dried and cut to a suitable shape for fitting over the viscous Aquacryl 90 solution in the mold. In certain embodiments, the Aquacryl 80 layer may include a reinforcing member which was included during solvent casting. In other embodiments, the reinforcing member may be placed over the viscous solution in the mold prior to placing the preformed more rigid layer in the mold. After the more rigid layer is placed over the viscous solution, additional viscous polymer solution is added to the mold until a desired thickness is reached. Another preformed, dried hydrogel layer, with or without the reinforcing member, is placed over the viscous solution, followed by another layer of viscous polymer solution. The process may be repeated until any desired number of layers is formed. The order of layering may be varied to suit particular applications. After the last layer is applied, the mold is closed and placed in water for solvent exchange. For example, the sodium thiosulfate solution diffuses out and is replaced with water, causing the viscous solution to coagulate. In the case of successive layers of Aquacryl 90 and Aquacryl 80, the layers adhere to each other without the need for any adhesives. In certain embodiments, the interface between the Aquacryl 90 layers and the Aquacryl 80 layers is blurred by comingling of the polymers during the manufacturing process, leading to a gradual transition from layer to layer. In other embodiments, adhesives such as polyurethanes or cyanoacrylates are used to bond the layers together.

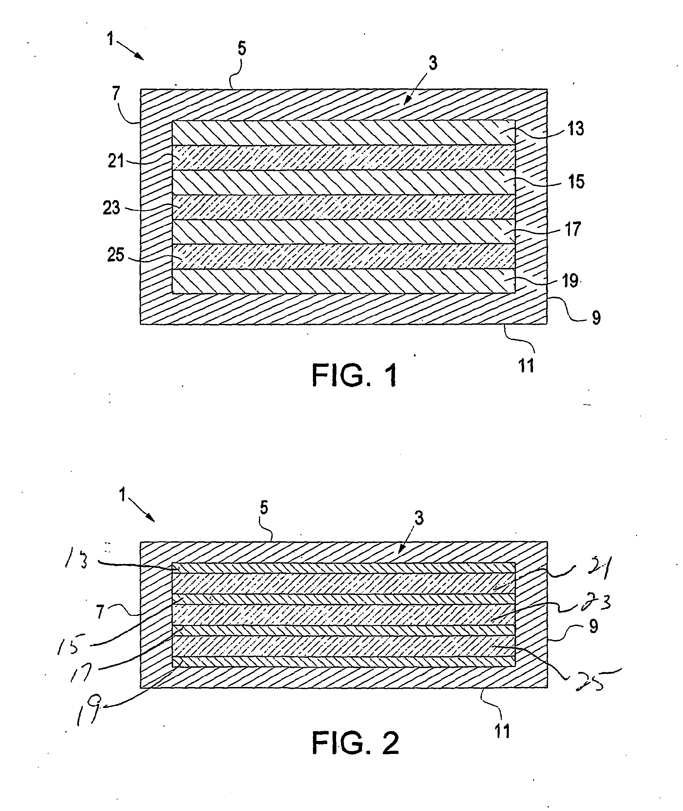

The invention is further illustrated in the following examples, which do not limit the scope of its' applicability, however. Fig. 1 shows a schematic cross-section of a fully hydrated prosthetic and Fig. 2 shows a cross-section of a dehydrated prosthetic.

### Example 1:

A prototype of the NP prosthetic was prepared using an AQUACRYL type hydrogel.

AQUACRYL is a copolymer, prepared by a partial alkaline hydrolysis of polyacrylonitrile (PAN) in the presence of sodium thiocyanate (NaSCN). The resulting hydrolysis product is a multi-block acrylic copolymer, containing alternating hydrophilic and hydrophobic blocks. Hydrophilic blocks contain acrylic acid, acrylamidine, and acrylamide. The hydrophobic blocks are formed by the remaining sequences of unreacted acrylonitrile units. The composition of the hydrolysis product varies with the type of AQUACRYL material, and depends on reaction conditions and the conversion of the hydrolytic reaction. The composition and basic properties of the two standard types used in this example are as follows:

| Copolymer composition (% mol) | **AQUACRYL 90** | **AQUACRYL 80** |
|---|---|---|
| Acrylonitrile units | 55 | 79 |
| Acrylic acid units | 30 | 14 |
| Acrylamide units | 9 | 4 |
| Acrylamidine units | 6 | 3 |

### Essential characteristics of the coagulate

### Equilibrium in pure water

| Copolymer composition (% molar) | **AQUACRYL 90** | **AQUACRYL 80** |
|---|---|---|
| Liquid content (% by weight) | 99 | 90 |
| Tensile strength (kg.cm⁻²) | <0.1 | 7 |

### Equilibrium in an isotonic solution

| (0.9% NaCl in water) | | |
|---|---|---|
| Copolymer composition (% molar) | **AQUACRYL 90** | **AQUACRYL 80** |
| Liquid content (% by weight) | 90 | 80 |
| Tensile strength (kg.cm⁻²) | 6 | 17 |

AQUACRYL is supplied in the form of a 10% (by weight) polymer solution dissolved in a sodium thiocyanate solution (55% aqueous NaSCN by weight). AQUACRYL can be poured directly from such solution onto glass or plastic substrates in order to form hydrogel foil or plate shapes, or various shapes in semi-enclosed or porous molds. The gelling of the solution occurs through the so-called coagulation, i.e. the replacement of NaSCN by water.

The prosthetic is manufactured using several steps. The first step forms prefabricated soft and rigid layers which will be assembled into the final configuration in subsequent steps. Individual steps can be described as follows:

### The preparation of non-reinforced hydrogel foils

The AQUACRYL solution was poured onto a level glass plate, and spread out into desired thickness using a strip whose height above the glass surface was adjustable using thickness control elements. The solution was carefully covered with water and in about 10 minutes it was transferred into a wash basin filled with an excess of an isotonic solution (0.9% NaCl in water by weight). The coagulated foils were washed out in fresh isotonic solution and placed in a plasticizing solution, which contains 12.5% glycerine and 87.5 % isotonic NaCl solution (by weight). The foils remain in this solution for 24 hours.

### The preparation of reinforced hydrogel foils

Round stretching frames 15 cm in diameter were stretched with knit polyester netting supplied by the Knitting Research Institute a.s. in Brno. The stretched net was carefully placed on top of an aquacryl solution layer of a desired thickness located on top of a glass plate. The depth of immersion of the net into the aquacryl solution was controlled by spacers placed underneath the frame. The solution within the stretch frame was carefully covered with water, and in 10 minutes was transferred into a wash basin filled with an excess of an isotonic solution (0.9% NaCL in water by weight). The coagulated foils in the frames were washed out in fresh isotonic solution. They were then placed in the plasticizing solution formed by 12.5% glycerine and 87.5% isotonic NaCl solution (by weight), where they remained for 24 hours.

### Dehydration of hydrogel foils

The non-reinforced foils were removed from the plasticizing bath and the excess liquid was removed using a filter paper. Thus dried off foils were supported by polyester netting on both sides and stretched within the round stretch flames. The frames were secured around the perimeter using clamps to prevent the material from slipping out and placed in a dryer preheated to 65°C for two hours. Then they were removed and cooled to a room temperature at a relative humidity of 30-60%. Prior to further processing the foils were stored while stretched on frames in closed PE bags.

The reinforced foils were dried using the same method of being stretched onto the frames in which they were made. They were secured with clamps around the perimeter to prevent the material from slipping out during contraction and drying.

Dehydrated foils plasticized with glycerin hold their shape at room temperature. When immersed in an isotonic solution, such as plasma, they swell only in thickness, while their base shape does not change. This phenomenon of frozen shape memory in the plasticized state is specific for this type of hydrogel, and is also well suited for this application. -

The dehydrated plasticized foils were cut into elliptical shapes 30 mm long and 15 mm wide, with two holes 3mm in diameter located in the 1/3 and 2/3 of the main axis.

### Assembling the nucleus of the prosthesis

A stainless steel apparatus was constructed using two base plates and two connecting guide rails. Each base plate has an elliptical shape 34 mm long and 17 mm wide, with circular openings 3 mm in diameter centered on the main axis and located 5 mm from the center. Each plate is 5 mm thick. Two stainless steel guide rails, 2.95 mm in diameter, are 25 mm long.

The guide rails are inserted into one base plate, and the individual layers cut from the dehydrated, plasticized aquacryl foil are threaded onto them. Their type and order varies according to the intended prosthetic configuration and it's intended final mechanical properties.

As explained above, the desired characteristics are achieved by alternating soft and rigid layers.

In this example we manufactured 4 types of layers:

| **Layer** | **Hydrogel** | **Polyester netting reinforcement** |
|---|---|---|
| A | AQUACRYL 90 | No |
| B | AQUACRYL 90 | Yes |
| C | AQUACRYL 80 | No |
| D | AQUACRYL 80 | Yes |

Layers can be assembled in various orders, such as:
(1) B-A-B-A-B-A-B
(2) A-B-A-A-B-A-A-B-A
(3) A-D-A-D-A-D-A
(4) D-C-A-C-D-C-A-C-D-C-A-C-D
*(5)* D-C-D-C-A-C-D-C-A-A-C-D-C-A-C-C
(7) A-C-A-C-A-C-A-C-A
(8) C-D-C

As apparent above, foils B and D with an embedded net will always act as a rigid layer. Foil A made of the non-reinforced AQUACRYL 90 (AQC 90) will act as a soft layer. The non-reinforced foil C made of AQUACRYL 80 (AQC 80) can act as a rigid layer (such as in configuration 7) or as soft layers (config. 8), or as transitional layers between the soft and rigid type (for example, config. 4 and 5). The thickness of the individual soft or rigid layers can change according to the number of foils used and their thickness. This shows that this manufacturing method is quite flexible, and can be used to achieve various characteristics without changing the basic approach or product.

Mutual cross-linking of individual layers can be accomplished by using appropriate adhesives, which can be either reactive or solvent-based. Of the reactive ones, cyanoacrylates or polyurethanes with free isocyanate groups could be used, especially ones which form elastic and solid sub-layers. Of the solvent-based ones, the best ones are aqueous sodium thiocyanate, possibly in combination with potassium and calcium thiocyanate. Other suitable adhesives are also the solutions of AQUACRYL and other related acrylic copolymers.

### The method used in this example is as follows:

Aquacryl layers are loosely threaded onto guide rails in the desired sequence (such as A-D-A-D-A-A-D-A-A-D-A-D) so there is free space between them, and they are secured with the other base plate on the other side. The whole configuration is briefly immersed in a 55% NaSCN in water (by weight), and placed inside a press, where the excess of the liquid is expressed using a force of about 10 kg. The configuration is left under this weight for about 30 minutes, after which the nucleus is removed from the guide rails.

The composite nucleus is encapsulated in AQUACRYL 90 in the next step.

### Nucleus encapsulation

Once the layers are glued together, the resulting nucleus is allowed to swell in a physiological solution until it reached equilibrium. The swollen nucleus is patted dry, and immersed into a 55% NaSCN (by weight) for a period of time. Thus prepared nucleus is then placed into a form with porous walls, or semi-permeable walls (permeable to low molecular weight substances), and embedded in a AQC 90 solution so that a contiguous layer of AQC 90 is formed around the whole nucleus. Once the form is closed and the excess AQC 90 solution is expressed, the form is placed in a water bath where the AQC 90 coagulation will occur over a period of approximately 15 hours. Once the form is removed from the bath and the form is disassembled, the finished prosthetic is further washed in the waster to eliminate the remaining NaSCN. Then it is washed in a physiological solution until equilibrium is reached.

The resulting product is depicted on a schematic drawing in Fig. 1, where A represents AQUACRYL 90 soft hydrogel layers, D represents AQUACRYL 80 rigid hydrogel layers reinforced with a polyester net, and E represents the outer AQUACRYL 90 sheath surrounding the prosthetic.

### Anisotropic prosthesis dehydration

Once an equilibrium water concentration is achieved, the prosthesis is dried while weighed down. The weight is applied in a direction tangent to the plane of the nucleus which is located in a tube-like form of an elliptical cross-section. The internal dimensions of the form correspond to the dimensions of the nucleus' base shape. The direction of the load, selection of appropriate load level, and the placement of the disc in the form during drying result only the height of the prosthesis changing, but the base shape and dimensions staying the same. Drying in the air occurs for several days, after which the disk is finished in a dryer at 70 °C under steady load. The result of the dehydration process is a flat elliptical disc made of a hard xerogel, depicted schematically in Fig. 2.

### Sterilization of the prosthesis

The dried disc is wrapped in a semi-permeable sack (a wrapper used for gas or steam sterilization) and sterilized using gamma radiation. Once sterilized, the wrapper is exposed to a high relative humidity until the disc absorbs approximately 20% of its weight in water, and becomes plastic and bendable. Once in this state, it is enclosed in a water-tight wrapper and stored under controlled conditions.

The implantation can be performed by inserting a rolled-up dehydrated prosthesis through an appropriate channel into the cavity created by the removal of the NP or its part. It is advisable to fill or flush the rest of the cavity with an appropriate physiologically harmless isotonic solution which will speed up the unfurling and swelling of the prosthesis in situ.

### EXAMPLE 2

The dehydrated and radiation sterilized elliptical disc from Example 1 is partially rehydrated using water vapor. Then it is heated up to 60°C and rolled under sterile conditions into a roll of about 7.5 mm in diameter, and chilled to a temperature of +4 °C. The rolled-up elliptical disc is then rinsed with aqueous ethanol for additional sterilization, dried off, and enclosed in sterile packaging. During implantation the rolled-up prosthesis is inserted through a channel into the intravertebral space and oriented appropriately, after which a sterile physiological solution at body temperature is injected into the space. Once heated up to body temperature, the prosthetic will unfurl from the rolled-up to a flat state. It will swell by absorbing water, and will expand in the axial direction relative to the spine. The speed of unfurling of the prosthetic and its subsequent expansion is controlled by the concentration of water in the hydrogel after its partial rehydration.

### EXAMPLE 3

The prosthetic can also be manufactured by using other materials than those described in examples 1 and 2. Elliptical profiles sized 30 x 17 mm are cut out of an open-cell polyurethane foam. One polyurethane foam type (A) is soft, yielding, with density of about 0.03 g.cm⁻³. The other foam type (B) is semi-rigid with a density of about 0.15 g.cm³.

The profiles are saturated by an AQUACRYL 90 solution from Example 1, and then they are stacked in a B-A-B-A-B order, enclosed in an encapsulation form from Example 1, and further processed by coagulation, washing, dehydration, and sterilization as in Example 1.

The prosthetics described in this invention can be used in the medical industry.

The above description sets forth preferred embodiments and examples. It should be understood that those skilled in the art will envision modifications of the embodiments and examples that, although not specifically stated herein, are still within the spirit and scope of the appended claims. For example, although the layers have been described as being substantially parallel, it is contemplated that in certain embodiments the individual layers are not uniformly thick, i.e. one side of a layer may be thicker than the other to form a cross-sectional wedge-shape. An adjacent layer may form a complementary shape such that the two layers fit together to form a substantially cylindrical shape. In addition, the reinforcing member, rather than being embedded in a layer, may be positioned between two layers.

## Claims

1. A prosthesis (1) for replacement of at least a part of the nucleus of an intravertebral disc, which comprises:
soft layers (13, 15, 17) of an elastically deformable hydrogel and at least one rigid layer (21, 23, 25), said rigid layer (21, 23, 25) having less compressibility than said soft layers (13, 15, 17) and firmly attached to them
**characterized in that**
there are at least two essentially parallel soft layers (13, 15, 17)
and the rigid layer (21, 23, 25) is adjacent to the soft layers (13, 15, 17) and parallel to them.

2. The prosthesis (1) according to Claim 1 wherein the soft layers (13, 15, 17) have the same thickness and composition.

3. The prosthesis (1) according to Claim 1 wherein there is more than one rigid layer and the rigid layers have the same thickness and composition.

4. The prosthesis (1) according to Claim 1 wherein the number of soft layers (13, 15, 17) is one more than the number of rigid layers (21, 23, 25).

5. The prosthesis (1) according to Claim 1 wherein there are at least three soft layers (13, 15, 17).

6. The prosthesis (1) according to Claim 1 wherein at least one of said at least one rigid layer (21, 23, 25) is reinforced by a reinforcement component selected from the group consisting of textile fibers, a perforated polymer foil or a combination of the two.

7. The prosthesis (1) according to Claim 6 wherein textile fibers are the reinforcing component and have a configuration selected from the group consisting of a knit net, a non-woven textile, or a woven lattice placed parallel to the firm layer surface.

8. The prosthesis (1) according to Claim 1 wherein the elastically deformable hydrogel in a state of maximum swelling has a water content of more than 75% by weight.

9. The prosthesis (1) according to Claim 1 wherein the elastically deformable hydrogel in a state of maximum swelling has a water content of more than 90% by weight.

10. The prosthesis (1) according to Claim 1 wherein the hydrogel is selected from a group consisting of partially hydrolyzed polyacrylonitrile, partially aminolyzed polyacrylonitrile, crystallized polyvinylalcohol, hydrophilic polyurethane, and a hydrogel derived from agar.

11. The prosthesis (1) according to Claim 1 wherein the rigid layers (21, 23, 25) contain a hydrogel of the same type as the hydrogel in the soft layers (13, 15, 17), but with a different water content.

12. The prosthesis (1) according to Claim 1 wherein the individual layers are firmly attached by mutual cross-linking of both layers.

13. The prosthesis (1) according to Claim 1 wherein the volume of said prosthesis (1) prior to implantation is lessened by at least partial dehydration.

14. The prosthesis (1) according to Claim 13 wherein said at least partially dehydrated prosthesis being in the flat state, where the base contour and surface are preserved, and the thickness of the layers is diminished in a ratio to the volumetric change caused by the dehydration.

15. The prosthesis (1) according to Claim 1 wherein said prosthesis (1) is plasticized by water, by a physiologically harmless plasticizer mixable with water, or a combination of such plasticizers.

16. The prosthesis (1) according to Claim 15 wherein the softening point of the plasticized prosthetic is between -5°C to 40°C.

17. A method of production of a prosthesis (1) according to claim 1, which comprises:
prefabricating soft (13, 15, 17) and rigid (21, 23, 25) layers;
stacking at least two prefabricated soft (13, 15, 17) and at least one prefabricated rigid layer (21, 23, 25) in a substantially parallel fashion into their final form; and,
permitting the layers to firmly connect to one another by mutual interaction.

18. The method of prosthesis (1) production according to Claim 17 wherein at least one type of said layers is prefabricated in the dehyrated state, which is done by dehydrating stretched foil in an apparatus preventing its contraction and thereby decreasing its area.

19. The method of prosthesis (1) production according to Claim 17 further comprising an at least partial dehydration of the prosthesis under pressure applied in a direction tangent to the planes of the layers.

20. The method of prosthesis (1) production according to Claim 19 wherein the prosthesis (1), while in the state of almost full dehydration, is sterilized using ionizing radiation or a gaseous chemical agent, after which it is partially rehydrated within a sterile wrapper using water vapor.

## Patentansprüche

1. Prothese (1) zum Ersatz wenigstens eines Teils des Kerns einer Bandscheibe, die Folgendes umfasst:
weiche Schichten (13, 15, 17) aus einem elastisch verformbaren Hydrogel und mindestens eine feste Schicht (21, 23, 25), wobei die feste Schicht (21, 23, 25) eine geringere Kompressibilität besitzt als die weichen Schichten (13, 15, 17) und fest daran haftet,
**dadurch gekennzeichnet, dass**
es mindestens zwei im Wesentlichen parallele weiche Schichten (13, 15, 17) gibt
und die feste Schicht (21, 23, 25) an die weichen Schichten (13, 15, 17) angrenzt und parallel dazu ist.

2. Prothese (1) nach Anspruch 1, wobei die weichen Schichten (13, 15, 17) dieselbe Dicke und Zusammensetzung haben.

3. Prothese (1) nach Anspruch 1, wobei es mehr als eine feste Schicht gibt und die festen Schichten dieselbe Dicke und Zusammensetzung haben.

4. Prothese (1) nach Anspruch 1, wobei die Zahl weicher Schichten (13, 15, 17) eins größer ist als die Zahl fester Schichten (21, 23, 25).

5. Prothese (1) nach Anspruch 1, wobei es mindestens drei weiche Schichten (13, 15, 17) gibt.

6. Prothese (1) nach Anspruch 1, wobei mindestens eine von der mindestens einen festen Schicht (21, 23, 25) durch eine Verstärkungskomponente verstärkt ist, die ausgewählt ist aus der aus Textilfasern, einer perforierten Polymerfolie oder einer Kombination der beiden bestehenden Gruppe.

7. Prothese (1) nach Anspruch 6, wobei Textilfasern die Verstärkungskomponente sind und eine Gestalt besitzen, die ausgewählt ist aus der aus einem Stricknetz, einem Vliesstoff oder einem parallel zur Oberfläche der festen Schicht angeordneten gewebten Gitter bestehenden Gruppe.

8. Prothese (1) nach Anspruch 1, wobei das elastisch verformbare Hydrogel in einem Zustand maximaler Quellung einen Wassergehalt von mehr als 75 Gew.-% hat.

9. Prothese (1) nach Anspruch 1, wobei das elastisch verformbare Hydrogel in einem Zustand maximaler Quellung einen Wassergehalt von mehr als 90 Gew.-% hat.

10. Prothese (1) nach Anspruch 1, wobei das Hydrogel ausgewählt ist aus einer aus teilweise hydrolysiertem Polyacrylnitril, teilweise aminolysiertem Polyacrylnitril, kristallisiertem Polyvinylalkohol, hydrophilem Polyurethan und einem aus Agar gewonnenen Hydrogel bestehenden Gruppe.

11. Prothese (1) nach Anspruch 1, wobei die festen Schichten (21, 23, 25) ein Hydrogel derselben Art wie das Hydrogel in den weichen Schichten (13, 15, 17), aber mit einem anderen Wassergehalt, enthalten.

12. Prothese (1) nach Anspruch 1, wobei die einzelnen Schichten durch gegenseitige Vernetzung beider Schichten fest aneinander haften.

13. Prothese (1) nach Anspruch 1, wobei das Volumen der Prothese (1) vor Implantation durch wenigstens teilweise Dehydrierung verringert wird.

14. Prothese (1) nach Anspruch 13, wobei sich die wenigstens teilweise dehydrierte Prothese im flachen Zustand befindet, wo Basiskontur und Grundfläche bewahrt bleiben und die Dicke der Schichten im Verhältnis zu der durch die Dehydrierung bedingten Volumenänderung verringert ist.

15. Prothese (1) nach Anspruch 1, wobei die Prothese (1) durch Wasser, einen mit Wasser mischbaren physiologisch harmlosen Weichmacher oder eine Kombination solcher Weichmacher weichgemacht wird.

16. Prothese (1) nach Anspruch 15, wobei der Erweichungspunkt der weichgemachten Prothese zwischen -5°C und 40°C liegt.

17. Verfahren zur Herstellung einer Prothese (1) nach Anspruch 1 mit den folgenden Schritten:
Vorfertigen weicher Schichten (13, 15, 17) und fester Schichten (21, 23, 25);
Stapeln von mindestens zwei vorgefertigten weichen Schichten (13, 15, 17) und mindestens einer vorgefertigten festen Schicht (21, 23, 25) in einer im Wesentlichen parallelen Weise zu ihrer endgültigen Form; und
die Schichten sich durch gegenseitige Wechselwirkung fest miteinander verbinden lassen.

18. Verfahren zur Herstellung einer Prothese (1) nach Anspruch 17, wobei mindestens eine Art der genannten Schichten im dehydrierten Zustand vorgefertigt wird, was **dadurch** erfolgt, dass gedehnte Folie in einer Vorrichtung dehydriert wird, die ihre Kontraktion und **dadurch** eine Verringerung ihrer Fläche verhindert.

19. Verfahren zur Herstellung einer Prothese (1) nach Anspruch 17, das ferner eine wenigstens teilweise Dehydrierung der Prothese unter Druck umfasst, der in einer Richtung tangential zu den Ebenen der Schichten aufgebracht wird.

20. Verfahren zur Herstellung einer Prothese (1) nach Anspruch 19, wobei die Prothese (1) im Zustand einer fast vollständigen Dehydrierung mit Hilfe ionisierender Strahlung oder eines gasförmigen chemischen Stoffes sterilisiert wird, wonach sie in einer sterilen Hülle mittels Wasserdampf teilweise rehydriert wird.

## Revendications

1. Prothèse (1) pour le remplacement d'au moins une partie du noyau d'un disque intervertébral, qui comprend:
des couches molles (13, 15, 17) en un hydrogel élastiquement déformable et au moins une couche rigide (21, 23, 25), ladite couche rigide (21, 23, 25) ayant moins de compressibilité que lesdites couches molles (13, 15, 17) et étant solidement fixée à celles-ci,
**caractérisée en ce que**
il y a au moins deux couches molles essentiellement parallèles (13, 15, 17),
et la couche rigide (21, 23, 25) est adjacente aux couches molles (13, 15, 17) et parallèle à celles-ci.

2. Prothèse (1) selon la revendication 1, où les couches molles (13, 15, 17) ont la même épaisseur et composition.

3. Prothèse (1) selon la revendication 1, où il y a plus d'une couche rigide, et les couches rigides ont la même épaisseur et composition.

4. Prothèse (1) selon la revendication 1, où le nombre de couches molles (13, 15, 17) est un de plus que le nombre de couches rigides (21, 23, 25).

5. Prothèse (1) selon la revendication 1, où il y a au moins trois couches molles (13, 15, 17).

6. Prothèse (1) selon la revendication 1, où au moins une desdites au moins une couche rigide (21, 23, 25) est renforcée par un composant de renforcement sélectionné dans le groupe constitué de fibres textiles, d'une feuille de polymère perforée ou d'une combinaison des deux.

7. Prothèse (1) selon la revendication 6, où les fibres textiles sont le composant de renforcement et ont une configuration sélectionnée dans le groupe constitué d'un filet tricoté, d'un textile non-tissé ou d'une grille tissée placée parallèlement à la surface de couche solide.

8. Prothèse (1) selon la revendication 1, où l'hydrogel élastiquement déformable dans l'état du gonflement maximum possède une teneur en eau supérieure à 75% en poids.

9. Prothèse (1) selon la revendication 1, où l'hydrogel élastiquement déformable dans un état de gonflement maximum possède une teneur en eau supérieure à 90% en poids.

10. Prothèse (1) selon la revendication 1, où l'hydrogel est sélectionné dans un groupe consistant en polyacrylonitrile partiellement hydrolysé, polyacrylonitrile partiellement aminolysé, polyvinyl alcool cristallisé, polyuréthanne hydrophile et un hydrogel dérivé de l'agar-agar.

11. Prothèse (1) selon la revendication 1, où les couches rigides (21, 23, 25) contiennent un hydrogel du même type que l'hydrogel dans les couches molles (13, 15, 17), mais avec une teneur en eau différente.

12. Prothèse (1) selon la revendication 1, où les couches individuelles sont fixées solidement par une réticulation mutuelle des deux couches.

13. Prothèse (1) selon la revendication 1, où le volume de ladite prothèse (1) avant l'implantation est diminué par une déshydratation au moins partielle.

14. Prothèse (1) selon la revendication 13, où ladite prothèse au moins partiellement déshydratée étant dans l'état plat, où le contour de base et la surface sont préservés, et l'épaisseur des couches est diminuée en un rapport au changement volumétrique provoqué par la déshydratation.

15. Prothèse (1) selon la revendication 1, où ladite prothèse (1) est plastifiée par l'eau, par un plastifiant physiologiquement inoffensif pouvant être mélangé avec de l'eau ou en une combinaison de tels plastifiants.

16. Prothèse (1) selon la revendication 15, où le point de ramollissement de la prothèse plastifiée est entre -5°C à 40°C.

17. Procédé de production d'une prothèse (1) selon la revendication 1, qui comprend:
la préfabrication de couches molles (13, 15, 17) et rigides (21, 23, 25);
l'empilage d'au moins deux couches molles préfabriquées (13, 15, 17) et d'au moins une couche préfabriquée rigide (21, 23, 25) d'une manière sensiblement parallèle en leur forme finale; et
amener les couches à se lier solidement les unes aux autres par interaction mutuelle.

18. Procédé de production de prothèse (1) selon la revendication 17, où au moins un type desdites couches est préfabriqué dans l'état déshydraté, ce qui est effectué en déshydratant une feuille étirée dans un appareil empêchant sa contraction et diminuant ainsi sa zone.

19. Procédé de production de prothèse (1) selon la revendication 17, comprenant en outre une déshydratation au moins partielle de la prothèse sous pression appliquée dans une direction tangentielle aux plans des couches.

20. Procédé de production de prothèse (1) selon la revendication 19, où la prothèse (1), pendant qu'elle est dans l'état d'une déshydratation presque complète, est stérilisée en utilisant un rayonnement ionisant ou un agent chimique gazeux, après quoi elle est partiellement réhydratée dans une enveloppe stérile en utilisant de la vapeur d'eau.
